(19) Europäisches Patentamt
European Patent Office
Office européen des brevets

(11) Veröffentlichungsnummer: **0 007 007**
**B1**

(12) **EUROPÄISCHE PATENTSCHRIFT**

(45) Veröffentlichungstag der Patentschrift: **12.08.81**

(51) Int. Cl.³: **C 07 D 409/12,** A 61 K 31/415

(21) Anmeldenummer: **79101859.1**

(22) Anmeldetag: **11.06.79**

(54) **2-(N-(2'-Chlor-4'-methyl-thienyl-3')-N-(cyclopropylmethyl)-amino)-imidazolin-(2), dessen Säureadditionssalze, dieses enthaltende Arzneimittel und Verfahren zu seiner Herstellung.**

(30) Priorität: **10.07.78 DE 2830279**

(43) Veröffentlichungstag der Anmeldung: **23.01.80 Patentblatt 80/2**

(45) Bekanntmachung des Hinweises auf die Patenterteilung: **12.08.81 Patentblatt 81/32**

(84) Benannte Vertragsstaaten: **AT BE CH DE FR GB IT LU NL SE**

(56) Entgegenhaltungen:
**DE-A-2 116 903**
**DE-B2-1 941 761**
**DE-B2-2 308 883**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim (DE)**
Erfinder: **Köppe, Herbert, Dr., Neuweg 72, D-6507 Ingelheim (DE)**
Erfinder: **Kummer, Werner, Dr., Georg-Scheuing-Strasse 15, D-6507 Ingelheim (DE)**
Erfinder: **Kobinger, Walter, Prof. Dr., Belghofergasse 27, A-1120 Wien (AT)**
Erfinder: **Lillie, Christian, Dr., Hansi-Niese-Weg 12, A-1130 Wien (AT)**
Erfinder: **Pichler, Ludwig, Dr., Gusshausstrasse 24, A-1040 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

2-(N-(2′-Chlor-4′-methyl-thienyl-3′)-N-(cyclopropylmethyl)-amino)-imidazolin-(2), dessen Säureadditionssalze, dieses enthaltende Arzneimittel und Verfahren zu seiner Herstellung

Die Erfindung betrifft 2-[N-(2′-Chlor-4′-methyl-thienyl-3′)-N-(cyclopropylmethyl)-amino]-imidazolin-(2) der allgemeinen Formel

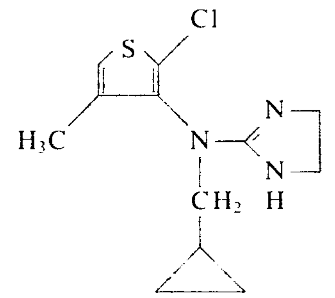

(I)

sowie dessen physiologisch verträgliche Säureadditionssalze mit wertvollen therapeutischen Eigenschaften.

Die Herstellung der neuen Verbindung der Formel I erfolgt durch Umsetzung von 2-(2′-Chlor-4′-methyl-thienyl-3′-amino]-imidazolin-(2) der Formel

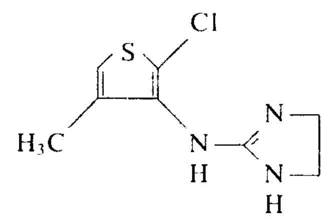

(II)

mit einem Cyclopropylmethylhalogenid der allgemeinen Formel

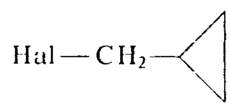

(III)

worin Hal ein Chlor-, Brom- oder Jodatom bedeutet.

Bei der Alkylierung der Verbindung der Formel II erfolgt die Substitution am Brückenstickstoffatom. Die Position des Substituenten läßt sich durch die NMR-Spektroskopie festlegen. (Vgl. H. Stähle und K.-H. Pook, Liebigs Ann. Chem. 751, 159 ff (1971).

Die Umsetzung erfolgt zweckmäßigerweise durch Erhitzen der Reaktionspartner — vorzugsweise in Gegenwart eines polaren oder unpolaren organischen Lösungsmittels — aus Temperaturen von 50 bis 100°C. Die speziellen Reaktionsbedingungen hängen in starkem Maße von der Reaktivität der Umsetzungsteilnehmer ab. Es empfiehlt sich, bei der Alkylierung das Halogenid im Überschuß anzuwenden und die Umsetzung in Gegenwart eines säurebindenden Mittels durchzuführen.

Die Ausgangsverbindung der Formel II ist in der DE-B-1 941 761 beschrieben.

Ausgangsverbindungen der Formel III lassen sich durch Halogenierung der zugrundeliegenden primären Alkohole darstellen.

Das erfindungsgemäße 2-Thienyl-amino-imidazolin-(2) der Formel I kann auf übliche Weise in seine physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neue Verbindung der Formel I und deren Säureadditionssalze wirken sehr stark bradycard und sind daher zur Therapie von Coronarerkrankungen geeignet. Die Beeinflussung der Herzfrequenz wurde an Kaninchen, Spinalratten und der inakten, narkotisierten Ratte untersucht. Die Dosierung liegt bei 0,1 bis 80 mg, vorzugsweise 1 bis 30 mg.

Die Verbindung der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die

üblicherweise verwendeten galenischen Hilfs-, Träger-, Spreng- oder Schmiermittel oder Substanzen zur Erzielung einer Depotwirkung Anwendung finden.

Folgende Beispiele erläutern die Erfindung.

Herstellungsbeispiel

2-[N-(Cyclopropylmethyl)-N-(2'-Chlor-4'-methyl-thienyl-3')-amino]-2-imidazolin

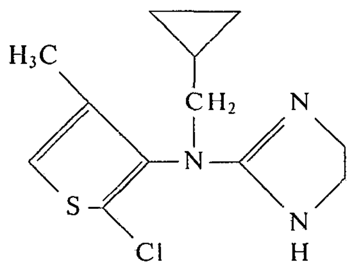

2,6 g (0,012 Mol) 2-(2'-Chlor-4'-methyl-thienyl-3'-amino)-imidazolin (Schmelzpunkt 152°C) werden zusammen mit 1,4 g (125%) Chlormethylcyclopropan in 20 ml absolutem Äthanol etwa 20 Stunden lang unter Rühren am Rückfluß erhitzt. Das Lösungsmittel wird hierauf im Vakuum abgezogen, der verbleibende Rückstand in verdünnter Salzsäure gelöst (gerade kongosauer) und die salzsaure Lösung zweimal mit je 50 ml Äther ausgeschüttelt (Ätherextrakte werden verworfen). Anschließend wird bei aufsteigenden pH-Werten (stufenweises Alkalisieren mit jeweils 0,25 ml 2 N Natronlauge bis zur deutlich alkalischen Reaktion) fraktioniert mit Äther extrahiert (etwa 15 Ätherfraktionen). Diejenigen Fraktionen, welche das neue Imidazolin-derivat in reiner Form enthalten (durch Dünnschichtchromatogramm-Kontrolle:

Fließmittelsystem: Benzol : Dioxan : Äthanol : konz. Ammoniak = 50 : 40 : 5 : 5;
Sichtbarmachung : Jodplatinat;
Träger: Kieselgel-G-Fertigplatten Merck Nr. 60 F 254:
Rf = 0,2 [Vergleich: Rf Tiamenidin = 0,6]),

werden vereinigt, über $MgSO_4$ getrocknet und der Äther im Vakuum abgezogen.

Ausbeute: 0,85 g (26,1% der Theorie).

Schmelzpunkt: 105—108°C.

Elementaranalyse

$C_{12}H_{16}ClN_3S$ (269.78)

| | C | H | Cl | N | S |
|---|---|---|---|---|---|
| Ber. | 53,42 | 5,98 | 13,14 | 15,57 | 11,89 |
| Gef. | 53,91 | 6,04 | 13,10 | 15,24 | 11,67 |

Formulierungsbeispiele

Beispiel A: Drageés

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummiarabicum dragiert werden

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung

Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche (für die benannten Vertragsstaaten BE, CH, DE, FR, GB, IT, LU, NL, SE)**

1. 2-[N-(2'-Chlor-4'-methyl-thienyl-3')-N-(cyclopropyl-methyl)-amino]-imidazolin-(2) der Formel

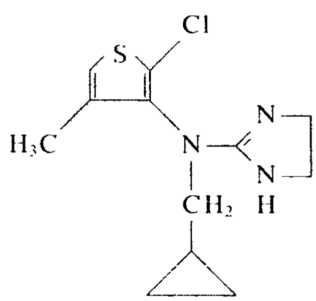

(I)

sowie dessen Säureadditionssalze.

2. Verfahren zur Herstellung der Verbindung der Formel I nach Anspruch 1 und von dessen Säureadditionssalzen, dadurch gekennzeichnet, daß man 2-(2'-Chlor-4'-methyl-thienyl-3'-amino)-imidazolin-(2) der Formel

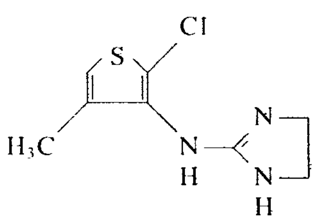

(II)

mit einem Cyclopropylmethyl-halogenid der allgemeinen Formel

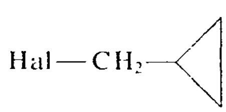

(III)

worin Hal ein Chlor-, Brom- oder Jodatom bedeutet, umsetzt und daß man gegebenenfalls das erhaltene Endprodukt in ein Säureadditionssalz überführt.

3. Verfahren nach Anspruch 2, dadurch gekennzeichnet, daß man die Umsetzung bei Temperaturen von 50 bis 100° C durchführt.

4. Verfahren nach Anspruch 2 und/oder 3, dadurch gekennzeichnet, daß man die Umsetzung in Gegenwart eines polaren oder unpolaren organischen Lösungsmittels durchführt.

5. Verfahren nach Anspruch 2 bis 4, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff die Verbindung der Formel I nach Anspruch 1 oder deren Säureadditionssalze enthalten.

7. Verfahren zur Herstellung von Arzneimitteln nach Anspruch 6, dadurch gekennzeichnet, daß man die Verbindung der Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verbindung der Formel I nach Anspruch 1 oder deren physiologisch verträgliche Säureadditionssalze zur Verwendung bei der Behandlung von Herz- und Coronarerkrankungen.

9. Verwendung der Verbindung der Formel I nach Anspruch 1 oder von deren physiologisch verträglichen Säureadditionssalzen zur Herstellung von Arzneimitteln für die Behandlung von Herz- und Coronarerkrankungen.

**Patentansprüche (für den benannten Vertragsstaat AT)**

1. Verfahren zur Herstellung von 2-[N-(2'-Chlor-4'-methyl-thienyl-3')-N-(cyclopropylmethyl)-amino]-imidazolin-(2) der Formel

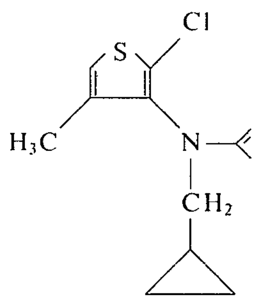

(I)

und von dessen Säureadditionssalzen, dadurch gekennzeichnet, daß man 2-(2'-Chlor-4'-methyl-thienyl-3'-amino)-imidazolin-(2) der Formel

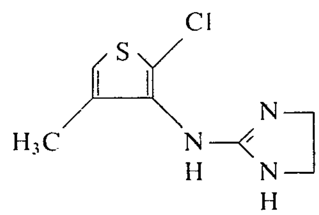

(II)

mit einem Cyclopropylmethyl-halogenid der allgemeinen Formel

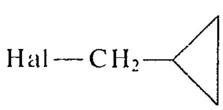

(III)

worin Hal ein Chlor-, Brom- oder Jodatom bedeutet, umsetzt und daß man gegebenenfalls das erhaltene Endprodukt in ein Säureadditionssalz überführt.

2. Verfahren nach Anspruch 1, dadurch gekennzeichnet, daß man die Umsetzung bei Temperaturen von 50 bis 100° C durchführt.

3. Verfahren nach Anspruch 1 und/oder 2, dadurch gekennzeichnet, daß man die Umsetzung in Gegenwart eines polaren oder unpolaren organischen Lösungsmittels durchführt.

4. Verfahren nach Anspruch 1 bis 3, dadurch gekennzeichnet, daß man die Umsetzung in Gegenwart eines säurebindenden Mittels durchführt.

5. Verfahren zur Herstellung von Arzneimitteln, dadurch gekennzeichnet, daß man die Verbindung der Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

6. Verwendung der Verbindung der Formel I nach Anspruch 1 oder von deren physiologisch verträglichen Säureadditionssalzen zur Herstellung von Arzneimitteln für die Bekämpfung von Herz- und Coronarerkrankungen.

**Claims (for the designated states BE, CH, DE, FR, GB, IT, LU, NL, SE)**

1. 2-[N-(2′-Chloro-4′-methyl-thienyl-3′)-N-(cyclopropyl-methyl)-amino]-imidazoline-(2) of the formula

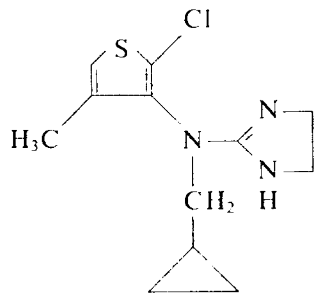

(I)

and the acid addition salts thereof.

2. Process for preparing the compouno of formula I according to claim 1 and the acid addition salts thereof, characterised in that 2-(2′-chloro-4′-methyl-thienyl-3′-amino)-imidazoline-(2) of formula

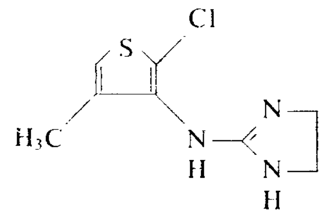

(II)

is reacted with a cyclopropylmethyl halide of general formula

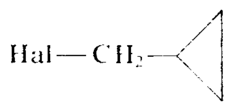

(III)

wherein Hal represents a chlorine, bromine or iodine atom, and, if required, the end product obtained is converted into an acid addition salt.

3. Process according to claim 2, characterised in that the reaction is carried out at temperatures of from 50 to 100° C.

4. Process according to claim 2 and/or 3, characterised in that the reaction is carried out in the presence of a polar or apolar organic solvent.

5. Process according to claims 2 to 4, characterised in that the reaction is carried out in the presence of an acid-binding agent.

6. Pharmaceutical preparations, characterised in that they contain, as active principle, the compound of formula I according to claim 1 or the acid addition salts thereof.

7. Process for preparing pharmaceutical compositions according to claim 6, characterised in that the compound of formula I according to claim 1 or the acid additions salts thereof is formulated with conventional galenic excipients, carriers, disintegrants or lubricants or substances for obtaining sustained release, to form tablets, capsules, suppositories, solutions or powders.

8. Compound of formula I according to claim 1 or the physiologically acceptable acid addition salts thereof for use in the treatment of heart and coronary diseases.

9. Use of the compound of formula I according to claim 1 or the physiologically acceptable acid addition salts thereof, for the preparation of pharmaceutical compositions for the treatment of heart and coronary diseases.

**Claims (for the designated state AT)**

1. Process for preparing 2-[N-(2′-chloro-4′-methyl-thienyl-3′)-N-(cyclopropylmethyl)-amino]-imidazoline-(2) of formula

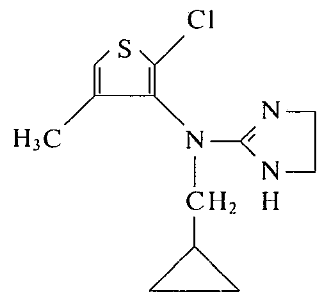

(I)

and the acid addition salts thereof, characterised in that 2-(2′-chloro-4′-methyl-thienyl-3′-amino)-imidazoline-(2) of formula

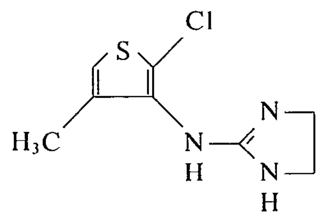

(II)

is reacted with a cyclopropylmethyl halide of general formula

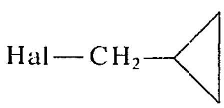

(III)

wherein Hal represents a chlorine, bromine or iodine atom, and if required, the end product obtained is converted into an acid addition salt.

2. Process according to claim 1, characterised in that the reaction is carried out at temperatures of from 50 to 100° C.

3. Process according to claim 1 and/or 2, characterised in that the reaction is carried out in the presence of a polar or apolar organic solvent.

4. Process according to claims 1 to 3, characterised in that the reaction is carried out in the presence of an acid-binding agent.

5. Process for preparing pharmaceutical compositions, characterised in that the compound of formula I according to claim 1 or the acid addition salts thereof is formulated with conventional galenic excipients, carriers, disintegrants or lubricants or substances for obtaining sustained release, to form tablets, capsules, suppositories, solutions or powders.

6. Use of the compound of formula I according to claim 1 or of the physiologically acceptable acid addition salts thereof for the preparation of pharmaceutical compositions for the treatment of heart and coronary diseases.

**Revendications (Pour les états contractants désignés BE, CH, DE, FR, GB, IT, LU, NL, SE)**

1. La 2-[N-(2′-chloro-4′-méthyl-thiényl-3′)-N-(cyclopropylméthyl)amino]imidazoline-(2) de formule

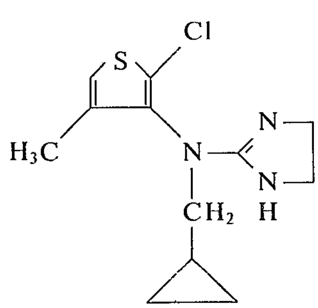

(I)

ainsi que ses sels d'addition avec des acides.

2. Procédé pour la préparation du composé de formule I selon la revendication 1 et de ses sels d'addition avec des acides, caractérisé en ce qu'on fait réagir la 2-(2'-chloro-4'-méthyl-thiényl-3'-amino)imidazoline (2) de formule

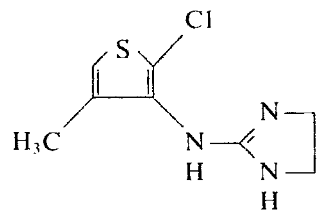

(II)

avec un halogénure de cyclopropylméthyle de formule générale

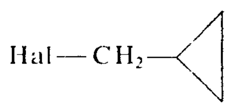

(III)

où Hal représente un atome de chlore, brome ou iode et en ce qu'éventuellement on transforme le produit final obtenu en un sel d'addition avec un acide.

3. Procédé selon la revendication 2, caractérisé en ce qu'on effectue la réaction à des températures de 50 à 100° C.

4. Procédé selon la revendication 2 et/ou 3, caractérisé en ce qu'on effectue la réaction en présence d'un solvant organique polaire ou non polaire.

5. Procédé selon la revendication 2 à 4, caractérisé en ce qu'on effectue la réaction en présence d'un agent fixant les acides.

6. Préparations pharmaceutiques, caractérisées en ce qu'elles contiennent, en tant que substance active, le composé de formule I selon le revendication 1 ou ses sels d'addition avec des acides.

7. Procédé de préparation des médicaments selon le revendication 6, caractérisé en ce qu'on formule le composé de formule I selon la revendication 1 ou ses sels d'addition avec des acides avec des Adjuvants, excipients, agents de désintégration ou lubrifiants ou respectivement substances pour obtenir un effet retard galéniques habituels, en comprimés, capsules, suppositoires, solutions ou poudres.

8. Composé de formule I selon la revendication 1 ou ses sels d'addition avec des acides physiologiquement supportables pour l'utilisation dans le traitement des maladies du coeur et des coronaires.

9. Utilisation du composé de formule I selon la revendication 1 ou de ses sels d'addition avec des acides physiologiquement supportables pour la préparation de médicaments pour le traitement des maladies du coeur et des coronaires.

**Revendications (pour l'état contractant désigné AT).**

1. Procédé pour la préparation de la 2-[N-(2'-chloro-4'-méthyl-thiényl-3')-N-(cyclopropylméthyl)-amino]-imidazoline-(2), de formule

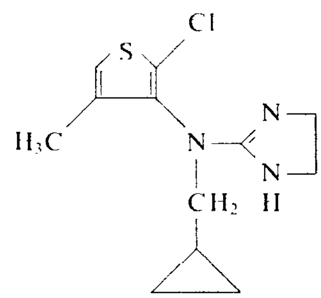

(I)

et de ses sels d'addition avec des acides, caractérisé en ce qu'on fait réagir la 2-(2'-chloro-4'-méthyl-thiényl-3'-amino)-imidazoline-(2) de formule

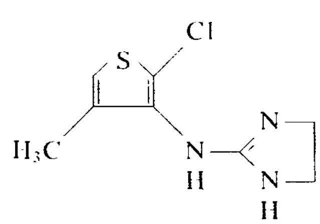

(II)

avec un halogénure de cyclopropylméthyle de formule générale

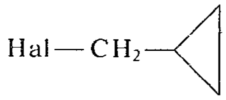

(III)

où Hal représente un atome de chlore, brome ou iode et en ce qu'éventuellement on transforme le produit final obtenu en un sel d'addition avec un acide.

2. Procédé selon la revendication 1, caractérisé en ce qu'on effectue la réaction à des températures de 50 à 100° C.

3. Procédé selon la revendication 1 et/ou 2, caractérisé en ce qu'on effectue la réaction en présence d'un solvant organique polaire ou non polaire.

4. Procédé selon la revendication 1 à 3, caractérisé en ce qu'on effectue la réaction en présence d'un agent fixant les acides.

5. Procédé pour la préparation de médicaments, caractérisé en ce qu'on formule le composé de formule I selon la revendication 1 ou ses sels d'addition avec des acides des adjuvants, excipients, agents de désintégration ou lubrifiants ou respectivement substances pour obtenir un effect retard galéniques habituels, en comprimés, capsules, suppositoires, solutions ou poudres.

6. Utilisation du composé de formule I selon la revendication 1 ou de ses sels d'addition avec des acides physiologiquement supportables pour la préparation de médicaments pour lutter contre les maladies du coeur et des coronaires.